# EUROPEAN PATENT SPECIFICATION

(11) **EP 0 926 129 B1**
(45) Date of publication and mention of the grant of the patent: **20.03.2002**
(21) Application number: 98122537.8
(22) Date of filing: 01.12.1998
(51) Int. Cl.: C07C 69/747, A01N 53/00

(54) **Ester compound and pesticide containing it**
Esterverbindung und diese enthaltendes Schädlingsbekämpfungsmittel
Composé ester et pesticide le contenant

(30) Priority: 03.12.1997 JP 33281597; 02.06.1998 JP 15273798
(43) Date of publication of application: 30.06.1999
(73) Proprietor: Sumitomo Chemical Company, Limited, Chuo-ku Osaka 541-8550 (JP)
(72) Inventor: Mori, Tatsuya, Toyonaka-shi, Osaka (JP); Matsuo, Noritada, Amagasaki-shi, Hyogo (JP)
(74) Representative: VOSSIUS & PARTNER

(56) References cited:
- EP-A- 0 638 543
- EP-A- 0 779 269
- KIRK A. SIMMONS ET AL.: "Predicting the Activity of Soil-Applied Insecticides from Their Physicochemical Properties" JOURNAL OF AGRICULTURAL AND FOOD CHEMISTRY., vol. 40, no. 8, August 1992, pages 1432-1436, XP002095886 WASHINGTON US

## Description

The present invention relates to a pyrethroid compound and a pesticide containing it.

Hitherto, it is known that chrysanthemic acid [3-(2-methyl-1-propenyl)-2,2-dimethylcyclopropanecarboxylic acid] esters whose alcohol components are 4-allyl-2,3,5,6-tetrafluorobenzyl alcohol and 2,3,5,6-tetrafluoro-4-methoxybenzyl alcohol have pesticidal activity (EP-A-31199). However, these ester compounds are not satisfactory for pesticidal active ingredient.

Moreover, it is known that 2,3,5,6-tetrafluoro-4-methylbenzyl(1R)-cis-3-(2-methyl-1-propenyl)-2,2-dimethylcyclopropanecarboxylate has pesticidal activity against soil insects (J. Agric. Food. Chem. (1992), 40(8), 1432). However, the compound has high toxicity for mammals. Therefore, it has a problem to be used as pesticide in safety, especially for household in a limited living space.

The present invention provides 2,3,5,6-tetrafluoro-4-methylbenzyl(1R)-trans-3-(2-methyl-1-propenyl)-2,2-dimethylcyclopropanecarboxylate (hereinafter referred to as "the present compound") shown by the formula (I): 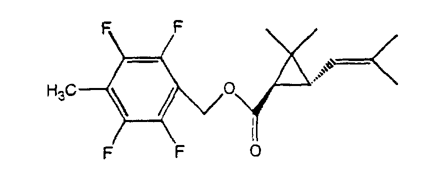 and a pesticide containing it as an active ingredient.

The present compound can be produced, for example, by reacting an alcohol compound shown by the formula (II): 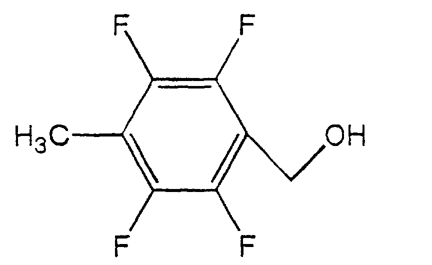 with (1R)-trans-3-(2-methyl-1-propenyl)-2,2-dimethylcyclopropanecarboxylic acid shown by the formula (III): 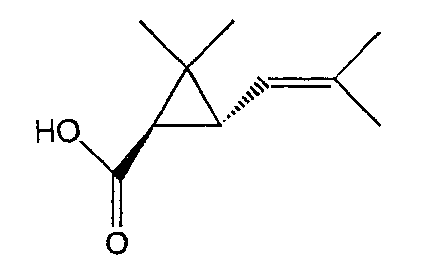 or its reactive derivatives (for example, acid halide, acid anhydride).

The reaction is usually carried out in the presence of a condensing agent or base in an inert solvent.

Examples of the condensing agent include dicyclohexylcarbodiimide (DCC) and 1-ethyl-3-(3-dimethylaminopropyl)carbodiimide hydrochloride (WSC).

Examples of the base include organic bases such as triethylamine, pyridine, N,N-diethylaniline, 4-dimethylaminopyridine and diisopropylethylamine.

Examples of the solvent to be used include hydrocarbons such as benzene, toluene and hexane; ethers such as diethyl ether and tetrahydrofuran; and halogenated hydrocarbons such as dichloromethane and 1,2-dichloroethane.

The reaction time is usually within a range from 5 minutes to 72 hours.

The reaction temperature is usually within a range from -20 °C to the boiling point of the solvent used for the reaction or a temperature up to 100 °C, preferably from -5 °C to the boiling point of the solvent used for the reaction or a temperature up to 100 °C.

The molar ratio of the alcohol compound shown by the formula (II) to the carboxylic acid shown by the formula (III) or the reactive derivative thereof to be used can be optionally set, but is advantageous to set an equimolar ratio or the ratio similar to the equimolar ratio.

The condensing agent or base can be used in an amount within a range from an equimolar amount to an excessive amount, preferably from an equimolar amount to 5 mols, based on 1 mol of the alcohol compound shown by the formula (II).

After the completion of the reaction, the reaction solution can be subjected to a usual work-up treatment such as extraction with organic solvent and concentration to give the present compound. If necessary, it may be purified by usual procedures such as chromatography and/or distillation.

The alcohol compound shown by the formula (II) and the carboxylic acid shown by the formula (III) can be prepared according to the method described in EP-A-31199.

Examples of noxious pests against which the present compound exhibits a control effect include the following arthropods:
Lepidoptera :
   E.g.Pyralidae such as *Chilo suppressalis* (rice stem borer), *Cnaphalocrocis inedinalis* (rice leafroller) and *Plodia interpunctella* (Indian meal moth); Noctuidae such as *Spodoptera litura* (tobacco cutworm), *Pseudaletia separata* (rice armyworm) and *Mamestra brassicae* (cabbage armyworm); Pieridae such as *Pieris rapae crucivora* (common cabbageworm); Tortricidae such as *Adoxophyes spp.;* Carposinidae; Lyonetiidae; Lymantriidae; Plusiinae; *Agrotis spp.* such as *Agrotis segetum* (turnip cutworm) and *Agrotis ipsilon* (black cutworm); *Helicoverpa spp.; Heliotis spp*. ; *Plutella xylostella* (diamondback moth); *Parnara guttata* (rice skipper); *Tinea pellionella* (casemaking clothes moth); and *Tineola bisselliella* (webbing clothes moth);
Diptera :
   E.g. *Culex spp.* such as *Culex pipiens pallens* (common mosquito) and *Culex tritaeniorhynchus*; *Aedes spp.* such as *Aedes aegypti* and *Aedes albopictus*; *Anopheles spp.* such as *Anopheles sinensis;* Chironomidae (midges); Muscidae such as *Musca domestica* (housefly), *Muscina stabulans* (false stablefly) and *Fannia canicularis* (little housefly); Calliphoridae; Sarcophagidae; Anthomyiidae such as *Delia platura* (seedcorn maggot) and *Delia antiqua* (onion maggot); Tephritidae (fluit flies); Drosophilidae; Psychodidae (moth flies); Simuliidae (black flies); Tabanidae; Phoridae; Stomoxyidae;and Ceratopogonidae (biting midges);
Dictyoptera :
   E.g. *Blattella germanica* (German cockroach); *Periplaneta fuliginosa* (smokybrown cockroach); *Periplaneta americana* (American cockroach); *Periplaneta brunnea* (brown cockroach); and *Blatta orientalis* (oriental cockroach);
Hymenoptera :
   E.g. Formicidae (ants); Vespidae (hornets); Bethylidae; and Tenthredinidae (sawflies) such as *Athalis rosae ruficornis* (cabbage sawfly);
Siphonaptera :
   E.g. *Ctenocephalides canis* (dog flea); *Ctenocephalides felis* (cat flea); and *Pulex irritans;*
Anoplura :
   E.g. *Pediculus humanus humanus* (body louse); *Pthirus pubis* (crab louse); *Pediculus humanus capitis* (head louse); and *Pediculus corporis;*
Isoptera :
   E.g. *Reticulitermes speratus*; and *Coptotermes formosanus;*
Hemiptera :
   E.g. Delphacidae (planthoppers) such as *Laodelphax striatellus* (small brown planthopper), *Nilaparvata lugens* (brown planthopper) and *Sogatella furcifere* (white backed rice planthopper); leafhoppers such as *Nephotettix cincticeps, Nephotettix virescens*; Aphididae (aphids); plant bugs; Aleyrodidae (whiteflies); scales; Tingidae (lace bugs); and Psyllidae;
Coleoptera (beetles) :
   E.g. *Attagenus unicolor japonicus* (black carpet beetle) and *Authrenus verbasci* (varied carpet beetle); corn rootworms such as *Diabrotica virgifera* (western corn rootworm)and *Diabrotica undecimpunctata howardi* (southern corn rootworm); Scarabaeidae such as *Anomala cuprea* (cupreous chafer) and *Anomala rufocuprea* (soybeen beatle); weevils such as *Sitophilus zeamais* (maize weevil), *Lissorhoptrus oryzophilus* (ricewater weevil), ball weevil and *Callosobruchus chinensis* (adzuki bean weevil); Tenebrionidae (darkling beetles) such as *Tenebrio molitor* (yellow mealworm) and *Tribolium castaneum* (red flour beetle); leaf beetles such as *Oulema oryzae* (rice leaf beetle), *Phyllotreta striolata* (striped flea beetle) and *Aulacophora femoralis* (cucurbit leaf beetle); Anobiidae; *Epilachna spp.* such as *Epilachna vigintioctopunctata* (twenty-eight-spotted ladybird); Lyctidae (powderpost beetles); Bostrychidae (false powderpost beetles); Cerambycidae; and *Paederus fuscipes* (robe beetle);
Thysanoptera :
   E.g. *Thrips palmi;* western flower thrips ; and *Thrips hawaiiensis;*
Orthoptera :
   E.g. Gryllotalpidae (mole crickets); and Acrididae (grasshoppers);
Acarina :
   E.g. Dermanyssidae such as *Dermatophagoides farinae* (American house dust mite) and *Dermatophagoides pteronyssinus;* Acaridae such as *Tyrophagus putrescentiae* (mold mite) and *Aleuroglyphus ovatus;* Glycyphagidae such as *Glycyphagus privatus, Glycyphagus domesticus* and *Glycyphagus destructor*; Cheyletidae such as *Chelacaropsis malaccensis* and *Cheyletus fortis;* Tarsonemidae; *Chortoglyphus spp.*; and *Haplochthonius simplex;*
   Tetranychidae such as *Tetranychus urticae* (carmine spider mite), *Tetranychus kanzawai* (Kanzawa spider mite), *Panonychus citri* (citrus red mite) and *Panonychus ulmi* (European red mite); Ixodidae such as *Boophilus microplus* and *Haemaphysalis longiconis.*

The pesticides in the present invention are for controlling or repelling pests, especially insects and acarina.

The present compound to be used as an active ingredient of a pesticide is usually formulated by mixing with a solid carrier, a liquid carrier, a gaseous carrier or bait, or is impregnated with a base material of a mosquito-coil or mosquito-mat for electric heating fumigation. The present compound is used as formulation such as oil solutions, emulsifiable concentrates, wettable powders, flowable formulations (e.g. aqueous suspension, aqueous emulsion), granules, dusts, aerosols, volatile formulations such as mosquito-coil, mosquito-mats for electric heater and liquid for electric heater, heating fumigants such as combustible fumigant, chemical fumigant and a porous ceramic fumigant, non-heating volatile formulations applied on resin or paper, fogging formulations, ULV formulations (formulations for ultra low volume application) and poisonous baits. Surfactants or other auxiliaries are added to the formulation if necessary.

These formulations include the present compound as an active ingredient in an amount of 0.001% to 95% by weight.

Examples of the solid carrier to be used for the formulation include fine powder or granules of clays (e.g. kaolin clay, diatomaceous earth, synthetic hydrated silicon oxide, bentonite, Fubasami clay, acid clay), talc, ceramics and other inorganic minerals (e.g. sericite, quartz, sulfur, active carbon, calcium carbonate and hydrated silicon oxice). Examples of the liquid carrier include water, alcohols (e.g. methanol and ethanol), ketones (e.g. acetone and methyl ethyl ketone), aromatic hydrocarbons (e.g. benzene, toluene, xylene, ethylbenzene and methylnaphthalene), aliphatic hydrocarbons (e.g. hexane, cyclohexane, kerosine and gas oil), esters (ethyl acetate and butyl acetate), nitriles (e.g. acetonitrile and isobutyronitrile), ethers (e.g. diisopropyl ether and dioxane), acid amides (e.g. N,N-dimethylformamide and N,N-dimethylacetamide), halogenated hydrocarbons (dichloromethane, trichloroethane and carbon tetrachloride), dimethyl sulfoxide and vegetable oils (e.g. soybean oil, cottonseed oil). Examples of the gaseous carrier or propellant to be used for the formulation include freon gas, butane gas, LPG (liquefied petroleum gas), dimethyl ether and carbon dioxide.

Examples of the surfactant include alkyl sulfates, alkylsulfonates, alkylarylsulfonates, alkyl aryl ethers, polyoxyethylenealkyl aryl ethers, polyethylene glycol ethers, polyhydric alcohol esters and sugar alcohol derivatives.

Examples of the sticking agents, the dispersing agent and other auxiliaries include casein, gelatin, polysaccharides (e.g. starch, gum arabic, cellulose derivatives and alginic acid), lignin derivatives, bentonite, sugars and synthetic water-soluble polymers (e.g. polyvinyl alcohol, polyvinylpyrrolidone and polyacrylic acid). Examples of the stabilizer include PAP (acid isopropyl phosphate), BHT (2,6-di-tert-butyl-4-methyphenol), BHA (mixture of 2-tert-butyl-4-methoxyphenol and 3-tert-butyl-4-methoxyphenol), vegetable oils, mineral oils, surfactants, fatty acids and esters of fatty acid.

An example of the base material of the mosquito-coil is a mixture of raw plant powder such as wood powder and Pyrethrum marc and a binding agent like Tabu powder (powder of *Machilus thunbergii*), starch or gluten.

An example of the base material of the mosquito-mat for electric heating fumigation is a plate of compacted fibrils of cotton linters or a mixture of pulp and cotton linters.

The base material of the combustible fumigant includes, for example, an exothermic agent (e.g. a nitrate, a nitrite, a guanidine salt, potassium chlorate, nitrocellulose, ethylcellulose and wood powder), a pyrolytic stimulating agent (e.g. an alkali metal salt, an alkaline earth metal salt, a dichromate and a chromate), an oxygen source (e.g. potassium nitrate), a combustion assistant (e.g. melanin and wheat starch), a bulk filler (e.g. diatomaceous earth) and a binding agent (e.g. synthetic glue).

The base material of the chemical fumigant includes, for example, an exothermic agent (e.g. an alkali metal sulfide, a polysulfide, a hydrogensulfide, a hydrated salt and calcium oxide), a catalytic agent (e.g. a carbonaceous substance, iron carbide and activated clay), an organic foaming agent (e.g. azodicarbonamide, benzenesulfonylhydrazide, N,N'-dinitrosopentamethylene tetramine, polystyrene and polyurethane) and a filler (e.g. natural or synthetic fibers).

Examples of the base material of the non-heating volatile agent include thermoplastic resins, filter paper and Japanese paper.

The base material of the poisonous baits includes a bait component (e.g. grain powder, vegetable oil, sugar and crystalline cellulose), an antioxidant (e.g. dibutylhydroxytoluene and nordihydroguaiaretic acid), a substance for preventing erroneous eating (e.g. red pepper powder) and an attractant (e.g. cheese flavor, onion flavor and peanut oil).

The flowable formulations (aqueous suspension or aqueous emulsion)are usually prepared by finely dispersing the present compound at a ratio of 1 to 75 % in water containing a 0.5 to 15 % dispersing agent, a 0.1 to 10 % suspension assistant (for example, protective colloid or a thixotropic agent and 0 to 10 % additives (e.g. an antifoamer, a rust preventive agent, a stabilizer, a developing agent, a penetrating assistant, antifreezing agent, a bactericide and a fungicide). The present compound may be dispersed in oil, in which the present compound is substantially insoluble, to form oil suspensions.

Examples of the protective colloid include gelatin, casein, gums, cellulose ethers and polyvinyl alcohol. Examples of the thixotropic agent include bentonite, aluminum magnesium silicate, xanthan gum and polyacrylic acid.

The formulations thus obtained are used as prepared or diluted with water and may be used simultaneously with another insecticide, another acaricide, a repellent or a synergist under non-mixed conditions or pre-mixed conditions.

Examples of the insecticides and acaricides include organophosphorus compounds such as fenitrothion [O,O-dimethyl O-(3-methyl-4-nitrophenyl) phosphorothioate], fenthion [O,O-dimethyl O-(3-methyl-4-(methythio)phenyl) phosphorothioate], diazinon [O,O-diethyl O-2-isopropyl-6-methylpyrimidin-4-yl phosphorothioate], chlorpyrifos [O,O-diethyl O-3,5,6-trichloro-2-pyridyl phosphorothioate], acephate [O,S-dimethyl acetylphosphoramidothioate], methidathion [S-2,3-dihydro-5-methoxy-2-oxo-1,3,4-thiadiazol-3-ylmethyl O,O-dimethyl phosphorodithioate], disulfoton[O,O-diethyl S-2-ethylthioethyl phosphorodithioate], DDVP [2,2-dichlorovinyl dimethyl phosphate], sulprofos [O-ethyl O-4-(methylthio)phenyl S-propyl phosphorodithioate], cyanophos [O-4-cyanophenyl O,O-dimethyl phosphorothioate], dioxabenzofos [2-methoxy-4H-1,3,2-benzodioxaphosphorin 2-sulfide], dimethoate [O,O-dimethyl S-(N-methylcarbamoylmethyl) dithiophosphate], phenthoate [ethyl 2-dimethoxyphosphinothioylthio(phenyl) acetate], malathion [diethyl(dimethoxyphosphinothioylthio)succinate], trichlorfon [dimethyl 2,2,2-trichloro-1-hydroxyethylphosphonate], azinphos-methyl [S-3,4-dihydro-4-oxo-1,2,3-benzotriazin-3-ylmethyl O,O-dimethyl phosphorodithioate], monocrotophos [dimethyl (E)-1-methyl-2-(methylcarbamoyl)vinyl phosphate] and ethion [O,O,O',O'-tetraethyl S,S'-methylene bis(phosphorodithioate)], carbamate compounds such as BPMC [2-sec-butylphenyl methylcarbamate], benfracarb [ethyl N-[2,3-dihydro-2,2-dimethylbenzofuran-7-yloxycarbonyl(methyl) aminothio]-N-isopropyl- β -alaninate], propoxur [2-isopropoxyphenyl N-methylcarbamate], carbosulfan [2,3-dihydro-2,2-dimethyl-7-benzo[b]furanyl N-dibuthylaminothio-N-methylcarbamate], carbaryl [1-naphthyl N-methylcarbamate], methomyl [S-methyl N-[(methylcarbamoyl) oxy]thioacetimidate], ethiofencarb [2-(ethylthiomethyl)phenyl methylcarbamate], aldicarb [2-methyl-2-(methylthio) propionaldehyde O-methylcarbamoyloxime], oxamyl[N,N-dimethyl-2-methylcarbamoyloxyimino-2-(methylthio)acetamide], fenothiocarb [S-4-phenoxybuthyl N,N-dimethylthiocarbamate], pyrethroid compounds such as etofenprox [2-(4-ethoxyphenyl)-2-methylpropyl 3-phenoxybenzyl ether], fenvalerate [(RS)-α-cyano-3-phenoxybenzyl(RS)-2-(4-chlorophenyl)-3-methylbutyrate], esfenvalerate [(S)- α-cyano-3-phenoxybenzyl (S)-2-(4-chlorophenyl)-3-methylbutyrate], fenpropathrin [(RS)-α -cyano-3-phenoxybenzyl 2,2,3,3-tetramethylcyclopropanecarboxylate], cypermethrin [(RS)- α -cyano-3-phenoxybenzyl (1RS)-cis,trans-3-(2,2-dichlorovinyl)-2,2-dimethyl-cyclopropanecarboxylate], permethrin [3-phenoxybenzyl (1RS)-cis,trans-3-(2,2-dichlorovinyl)-2,2-dimethylcyclopropane-carboxylate], cyhalothrin [(RS)-α-cyano-3-phenoxybenzyl (Z)-(1RS)-cis-3-(2-chloro-3,3,3-trifluoroprop-1-enyl)-2,2-dimethylcyclopropanecarboxylate], deltamethrin [(S)-α-cyano-3-phenoxybenzyl (1R)-cis-3-(2,2-dibromovinyl)-2,2-dimethylcyclopropanecarboxylate], cycloprothrin [(RS)- α -cyano-3-phenoxybenzyl(RS)-2,2-dichloro-1-(4-ethoxyphenyl) cyclopropanecarboxylate], fluvalinate [α-cyano-3-phenoxybenzyl N-(2-chloro- α, α, α -trifluoro-p-tolyl)-D-valinate], bifenthrin [2-methylbiphenyl-3-ylmethyl (Z)-(1RS)-cis-3-(2-chloro-3,3,3-trifluoroprop-1-enyl)-2,2-dimethylcyclopropanecarboxylate], 2-methyl-2-(4-bromodifluoromethoxyphenyl)propyl 3-phenoxybenzyl ether, tralomethrin [(S)- α -cyano-3-phenoxybenzyl (1R-cis)3-{(1RS)(1,2,2,2-tetrabromoethyl)}-2,2-dimethylcyclopropane-carboxylate], silafluofen [(4-ethoxyphenyl){3-(4-fluoro-3-phenoxyphenyl)propyl}dimethylsilane], d-phenothrin [3-phenoxybenzyl (1R-cis,trans)-chrysanthemate], cyphenothrin [(RS)-α-cyano-3-phenoxybenzyl (1R-cis,trans)-chrysanthemate], d-resmethrin [5-benzyl-3-furylmethyl (1R-cis,trans)-chrysanthemate], acrinathrin [(S)- α -cyano-3-phenoxybenzyl (1R/cis(Z))-2,2-dimethyl-3-{3-oxo-3-(1,1,1,3,3,3-hexafluoropropyloxy)propenyl}cyclopropanecarboxylate], cyfluthrin [(RS)- α -cyano-4-fluoro-3-phenoxybenzyl 3-(2,2-dichlorovinyl)-2,2-dimethylcyclopropanecarboxylate], tefluthrin [2,3,5,6-tetrafluoro-4-methylbenzyl (1RS-cis(Z))-3-(2-chloro-3,3,3-trifluoroprop-1-enyl)-2,2-dimethylcyclopropanecarboxylate], transfluthrin [2,3,5,6-tetrafluorobenzyl(1R-trans)-3-(2,2-dichlorovinyl)-2,2-dimethylcyclopropanecarboxylate], tetramethrin [3,4,5,6-tetrahydrophthalimidomethyl (1RS)-cis,trans-chrysanthemate], allethrin [(RS)-3-allyl-2-methyl-4-oxocyclopent-2-enyl (1RS)-cis,trans-chrysanthemate], prallethrin [(S)-2-methyl-4-oxo-3-(2-propynyl)cyclopent-2-enyl (1R)-cis,trans-chrysanthemate], empenthrin [(RS)-1-ethynyl-2-methyl-2-pentenyl (1R)-cis,trans-chrysanthemate], imiprothrin [2,5-dioxo-3-(prop-2-ynyl)imidazolidin-1-ylmethyl (1R)-cis,trans-2,2-dimethyl-3-(2-methylprop-1-enyl)cyclopropanecarboxylate], d-furamethrin [5-(2-propynyl)furfuryl (1R)-cis,trans-chrysanthemate] and 5-(2-propynyl)furfuryl 2,2,3,3-tetramethylcyclopropanecarboxylate, nitroimidazolidine derivatives, N-cyanoamidine derivatives such as N-cyano-N'-methyl-N'-(6-chloro-3-pyridylmethyl)acetamidine, chlorinated hydrocarbons such as endosulfan [6,7,8,9,10,10-hexachloro-1,5,5a,6,9,9a-hexahydro-6,9-methano-2,4,3-benzodioxathiepine oxide], γ-BHC [1,2,3,4,5,6-hexachlorocyclohexane] and 1,1-bis (chlorophenyl)-2,2,2-trichloroethanol, benzoylphenylurea compounds such as chlorfluazuron [1-(3,5-dichloro-4-(3-chloro-5-trifluoromethylpyridyn-2-yloxy)phenyl)-3-(2,6-difluorobenzoyl)urea], teflubenzuron [1-(3,5-dichloro-2,4-difluorophenyl)-3-(2,6-difluorobenzoyl)urea] and flufenoxuron [1-(4-(2-chloro-4-trifluoromethylphenoxy)-2-fluorophenyl)-3-(2,6-difluorobenzoyl)urea], thiourea derivatives such as diafenthiuron [N-(2,6-diisopropyl-4-phenoxyphenyl)-N'-tert-butylcarbodiimide], phenylpyrazole compounds, metoxadiazone [5-methoxy-3-(2-methoxyphenyl)-1,3,4-oxadiazol-2-(3H)-one], bromopropylate [isopropyl 4,4'-dibromobenzilate], tetradifon [4-chlorophenyl 2,4,5-trichlorophenyl sulfone], chinomethionat [S,S-6-methylquinoxaline-2,3-diyldithiocarbonate], pyridaben [2-tert-butyl-5-(4-tert-butylbenzylthio)-4-chloropyridazin-3(2H)-one], fenpyroximate [tert-butyl (E)-4-[(1,3-dimethyl-5-phenoxypyrazol-4-yl)methyleneaminooxymethyl]benzoate], tebufenpyrad [N-(4-tert-butylbenzyl)-4-chloro-3-ethyl-1-methyl-5-pyrazolecarboxamide], polynactins complex[tetranactin, dinactin and trinactin], pyrimidifen [5-chloro-N-[2-{4-(2-ethoxyethyl)-2,3-dimethylphenoxy}ethyl]-6-ethylpyrimidin-4-amine], milbemectin, abamectin, ivermectin and azadirachtin [AZAD].

Examples of the repellents are exemplified by 3,4-caranediol, N,N-diethyl-m-toluamide, 1-methylpropyl 2-(2-hydroxyethyl)-1-piperidinecarboxylate, p-menthane-3,8-diol and botanical essential oils (e.g. hyssop oil).

Examples of the synergists are exemplified by bis-(2,3,3,3-tetrachloropropane) (S-421), N-(2-ethylhexyl)bicyclo [2.2.1]hept-5-ene-2,3-dicarboximide (MGK-264) and α -[2-(2-butoxyethoxy)ethoxy]-4,5-methylenedioxy-2-propyltoluene (piperonylbutoxide).

When the present compound is applied as an active ingredient of pesticides for house-hold use or animal-health use, emulsifiable concentrates, wettable powders and flowable formulations are diluted with water to the concentration of 0.1 to 10000 ppm. Oil solutions, aerosols, fumigants, volatile agents, fogging agents, ULV formulations, poisonous baits and resin formulations are used as prepared.

The amount and concentration of application may be varied optionally according to the type of the formulations, time, place, and method of application, kind of noxious pests and damage.

The present compound can exhibit its superior pest-controlling effect under heating or non-heating volatilation. Therefore, it is especially useful as an active ingredient of household pesticide.

### Examples

The present invention will be further illustrated in detail by the production examples, formulation examples and biological tests, although the present invention is not limited in any sense to these examples.

The production example is shown as follows.

### production example 1

To a mixture solution of 1.78 g of 2,3,5,6-tetrafluoro-4-methylbenzyl alcohol, 0.87 g of pyridine and 20 ml of tetrahydrofuran, 2.06 g of (1R)-trans-3-(2-methyl-1-propenyl)-2,2-dimethylcyclopropanecarboxylic chloride was added under ice-cooling and the mixture was stirred for 8 hours at room temperature. The reaction mixture was poured into about 100 ml of ice-water and extracted with each 100 ml of ethyl acetate twice. The combined ethyl acetate was washed with saturated brine, dried over anhydrous sodium sulfate and concentrated under reduced pressure to give crude product, which was subjected to silica gel column chromatography to give 2.75 g of purified 2,3,5,6-tetrafluoro-4-methylbenzyl (1R)-trans-3-(2-methyl-1-propenyl)-2,2-dimethylcyclopropanecarboxylate, the present compound (yield 87%).
¹H-NMR (internal standard ; TMS, in CDCl₃) δ values (ppm) : 1.13(s, 3H), 1.30(s, 3H), 1.40(d, 1H), 1.71(brs, 6H), 2.08(dd, 1H), 2.28(brs, 3H), 4.88(m, 1H), 5.20(dd, 2H).

Next, formulation examples are described below. Parts represent parts by weight in the following examples.

### Formulation example 1 ; Emulusifiable concentrates

Twenty parts of the present compound are dissolves in 65 parts of xylene, mixed with 5 parts of Sorpol 3005X (surfactant ; trademark of Toho Chemical Co., Ltd.), and stirred sufficiently to give 20 % emulusifiable concentrates.

### Formulation example 2 ; Wettable powders

40 parts of the present compound are mixed first with 5 parts of Sorpol 3005X (described above)and then with 32 parts of Carplex #80 (fine powder of synthetic hydrated silicon oxide ; trademark of Shionogi & Co., Ltd.) and 23 parts of 300-mesh diatomaceous earth, and stirred with a blender to give 40 % wettable powders.

### Formulation example 3 ; Granules

1.5 parts of the present compound are mixed with 98.5 parts of AGSORB LVM-MS 24/48 (granular carrier of calcined monmorillonite having the particle diameter of 24 to 48 meshes provided by OIL DRI Corp.) sufficiently to give 1.5 % granules for each compound.

### Formulation example 4 ; Microcapsules

A mixture of 10 parts of the present compound, 10 parts of phenylxylylethane and 0.5 part of Sumidur L-75 (tolylenediisocyanate provided by Sumitomo Bayer Urethane Co., Ltd.) is added to 20 parts of a 10% aqueous solution of gum arabic, and stirred with a homomixer to give an emulsion having the mean particle diameter of 20 µm. The emulsion is further mixed with 2 parts of ethylene glycol and allowed to react on a water bath of 60 °C for 24 hours to give a microcapsule slurry.

A thicking agent is prepared by dispersing 0.2 part of xanthan gum and 1.0 part of Beagum R (aluminum magnesium silicate ; trademark of Sanyo Chemical Co., Ltd.) in 56.3 parts of ion-exchanged water.

42.5 parts of the above microcapsule slurry and 57.5 parts of the above thicking agent are mixed to give 10 % microencapsulated formulation.

### Formulation example 5 ; Flowable formulation

A mixture of 10 parts of the present compound and 10 parts of phenylxylylethane is added to 20 parts of a 10 % aqueous solution of polyethylene glycol and stirred with a homomixer to give an emulsion having the mean particle diameter of 3 µm.

A thicking agent is prepared by dispersing 0.2 part of xanthan gum and 1.0 part of Beagum R (aluminum magnesium silicate ; trademark of Sanyo Chemical Co., Ltd.) in 58.8 parts of ion-exchanged water.

40 parts of the above emulsion and 60 parts of the above thicking agent are mixed to give 10 % flowable formulation.

### Formulation example 6 ; Dusts

5 parts of the present compound are mixed with 3 parts of Carplex #80 (described above), 0.3 parts of PAP and 91.7 parts of 300-mesh talc, and stirred with a blender to give 5 % dusts.

### Formulation example 7 ; Oil solution

0.1 part of the present compound is dissolved in 5 parts of dichloromethane and mixed with 94.9 parts of deodorized kerosine to give 0.1 % oil solution.

### Formulation example 8 ; Oil-based aerosols

An aerosol vessel is filled with the solution obtained by dissolving 1 part of the present compound with 5 parts of dichloromethane and 34 parts of deodorized kerosine. The vessel is then equipped with a valve and 60 parts of propellant (liquefied petroleum gas) is charged through the valve into the aerosol vessel under pressure to give oil-based aerosols.

### Formulation example 9 ; Water-based aerosols

An aerosol vessel is filled with 50 parts of purified water and a mixture of 0.6 part of the present compound, 5 parts of xylene, 3.4 parts of deodorized kerosine and 1 part of Atmos 300 (emulsifier ; trademark of Atlas Chemical Co.). The vessel is then equipped with a valve and 40 parts of propellant (liquefied petroleum gas) is charged through a valve into the aerosol vessel under pressure to give water-based aerosols.

### Formulation example 10 ; Mosquito-coil

A solution prepared by dissolving 0.3 g of the present compound in 20 ml of acetone is homogeneously mixed with 99.7 g of a carrier for a mosquito-coil (mixture of Tabu powder, Pyrethrum marc and wood powder at the ratio of 4:3:3). After 120 ml of water is added, the mixture is kneaded sufficiently, molded and dried to give mosquito-coil.

### Formulation example 11 ; Mosquito-mat for electric heating fumigation

10 ml of solution is prepared by dissolving 0.8 g of the present compound and 0.4 g of piperonyl butoxide in acetone. 0.5 ml of the obtained solution is impregnated with a base material (a plate of compacted fibrils of a mixture of pulp and cotton linters : 2.5 cm × 1.5 cm × 0.3 cm) homogeneously to give mosquito-mat.

### Formulation example 12 ; Solution for electric heating fumigation

Three parts of the present compound is dissolved in 97 parts of deodorized kerosine. The obtained solution is charged in a vessel of polyvinyl chloride. In the vessel is inserted a porous absorptive wick which is inorganic powder solidified with a binder and then calcined, the upper portion of which wick can be heated with a heater, to give electric heating fumigation devices using a liquid.

### Formulation example 13 ; Fumigant

A solution prepared by dissolving 100 mg of the present compound in an appropriate amount of acetone is impregnated with a porous ceramic plate (4.0 cm × 4.0 cm × 1.2 cm) to give fumigant.

### Formulation example 14 ; Volatile agent

A solution prepared by dissolving 100 µ g of the present compound in an appropriate amount of acetone is applied onto filter paper (2.0 cm × 2.0 cm × 0.3 mm) and the acetone is vaporized to give volatile agent.

### Formulation example 15 ; Acaricidal sheet

An acetone solution containing the present compound is impregnated filter paper so that the concentration of the present compound is 1 g / 1 m² and the acetone is evaporated to give acaricidal sheet.

The present compound was tested as an active ingredient of a pesticide.

### Biological test 1 ; Insecticidal test against housefly

Filter paper of 5.5 cm in diameter was laid in the bottom of a polyethylene cup (diameter : 5.5 cm). After 0.7 ml of a 500 ppm aqueous emulsion obtained by diluting emulsifiable concentrate of the present compound prepared according to the formulation example 1 was dropped on the filter paper and approximately 30 mg of sucrose as bait was uniformly scattered. Ten female houseflies (*Musca domestica*), which were low sensitive to pyrethroids, were left in the cup with a cover. After one day, the mortality was examined. As a result, it was found that the present compound exhibited the mortality of 100 %. In contrast, the treatment of the emulsifiable concentrate without active ingredient gave 0 % of the mortality.

### Biological test 2 ; Insecticidal test against German cockroach

Filter paper of 5.5 cm in diameter was laid in the bottom of a polyethylene cup (diameter : 5.5 cm). After 0.7 ml of a 500 ppm aqueous emulsion obtained by diluting emulsifiable concentrate of the present compound prepared according to the formulation example 1 was dropped on the filter paper and approximately 30mg of sucrose as bait was uniformly scattered. Two male German cockroaches (*Blattella germanica*), which were low sensitive to pyrethroids, were left in the cup with a cover. After six days, the mortality was examined. As a result, it was found that the present compound exhibited the mortality of 100 %. In contrast, the treatment of the emulsifiable concentrate without active ingredient gave 0 % of the mortality.

### Biological test 3 ; Insecticidal test against common mosquito

0.7 ml of aqueous emulsion, obtained by diluting emulsifiable concentrate prepared for the present compound according to the formulation example 1, was added to 100 ml of ion-exchanged water (concentration of active ingredient : 3.5 ppm). Twenty last-instar larvae of common mosquitoes (*Culex pipiens pallens*) were left in the water. After one day, the mortality of the common mosquitoes was examined. The result was evaluated by the standard as follows.
**a** : 90 % or more mortality
**b** : 10 % or more, less than 90 % mortality
**c** : less than 10 % mortality
As a result, it was found that the present compound was evaluated as **a** and the treatment of the emulsifiable concentrate without active ingredient gave **c**.

### Biological test 4 ; Insecticidal test against housefly

An acetone dilution for the present compound was applied to 10 female houseflies (*Musca domestica*) at the back thoracic region (active ingredient : 5 µg/one housefly) and the houseflies were left with water and feed. After 24 hours, the percent moribund was examined (two replicates). As a result, it was found that the present compound exhibited the percent moribund of 100 %. In contrast, the treatment of acetone without active ingredient gave 0 % of the percent moribund.

### Biological test 5 ; Insecticidal test against housefly (volatilation at room temperature)

After 0.64 ml of a 0.05 (w/v) % acetone solution of the present compound was dropped into an aluminum plate (bottom diameter : 7 cm), acetone was air-dried. Ten female houseflies (*Musca domestica*) were left in a polyethylene cup (diameter : 9 cm ; depth : 4.5 cm) and the cup was sealed with a 16-mesh nylon net to prevent direct contact of the houseflies with the compound. The cup was placed upside down on the aluminum plate at 25 °C for 120 minutes. The cup was then removed from the aluminum plate, and water and feed were given to the houseflies. After 24 hours, the percent moribund was examined (two replicates). As a result, it was found that the present compound exhibited the percent moribund of 100 %. In contrast, the treatment of acetone without active ingredient gave 0 % of the percent moribund.

### Biological test 6 ; Insecticidal test against webbing clothes moth

The present compound diluted with acetone to a predetermined concentration was applied to ten middle instar larvae of webbing clothes moth (*Tineola bisselliella*) at the central part of the back so as to give the active ingredient in a dose of 3 µg / insect. A wool muslin fabric (2 cm × 2 cm in size) was given to the webbing clothes moths as the food. After 7 days, the mortality and the degree of damage of the wool muslin fabric by the moths were examined (two replicates). The damage was evaluated as follows:
+++ : severe damage
++ : heavy damage
+ : slight damage
- : no damage
As a result, it was found that the present compound exhibited the mortality of 100 % and - damage. In contrast, the treatment of acetone without active ingredient gave 0 % of the mortality and +++ damage.

### Biological test 7 ; Insecticidal test against webbing clothes moth (volatilation at room temperature)

A wool muslin fabric (2 cm × 2 cm in size) was placed on the bottom of a polyethylene cup (bottom diameter : 10 cm, opening part diameter : 12.5 cm, height : 9.5 cm, volume : 950 cm³ ). Ten middle instar larvae of webbing clothes moth (*Tineola bisselliella*) were put in the cup, the cup was covered up and the volatile agent prepared according to the formulation example 14 was hung from the cap in the interior of the cup. After standing at 25 °C for 1 week, the cup was opened, and the percent moribund and the degree of damage of the wool muslin fabric by the moths were examined (two replicates). The damage was evaluated as follows:
+++ : severe damage
++ : heavy damage
+ : slight damage
- : no damage
As a result, it was found that the present compound exhibited the percent moribund of 100 % and - damage. In contrast, the treatment of acetone without active ingredient gave 0 % of the percent moribund and +++ damage.

In this test, there was not found a change of color of filter paper or a unpleasant odor neither quickly, nor one week after treating.

### Biological test 8 ; Insecticidal test against common mosquito (volatilation at room temperature)

After 0.64 ml of a 0.05 (w/v) % acetone solution of the present compound (1) was dropped into an aluminum plate (bottom diameter : 7 cm), acetone was air-dried. Ten female common mosquitoes (*Culex pipiens pallens*) were left in a polyethylene cup (diameter : 9 cm ; depth : 4.5 cm) and the cup was sealed with a 16-mesh nylon net to prevent direct contact of the mosquitoes with the compound. The cup was placed upside down on the aluminum plate at 25 °C for 120 minutes. The cup was then removed from the aluminum plate, and water and feed were given to the mosquitoes. After 24 hours, the percent moribund was examined (two replicates). The same tests were conducted concerning to 4-allyl-2,3,5,6-tetrafluorobenzyl(1R)-trans-chrysanthemate (hereinafter referred to "compound A") and 2,3,5,6-tetrafluoro-4-methoxybenzyl (1R)-trans-chrysanthemate (hereinafter referred to "compound B"). The results are shown in table 1.

**[Table 1]**

| test area | dosage (mg/m²) | percent moribund (%) |
|---|---|---|
| the area treated the present compound | 0.625 | 70 |
| | 1.25 | 85 |
| the area treated compound A | 0.625 | 0 |
| | 1.25 | 15 |
| the area treated compound B | 0.625 | 0 |
| | 1.25 | 40 |
| the area treated acetone without active ingredient | - | 0 |
| | | |
| | | |

## Claims

1. 2,3,5,6-tetrafluoro-4-methylbenzyl(1R)-trans-3-(2-methyl-1-propenyl)-2,2-dimethylcyclopropanecarboxylate shown by the formula :

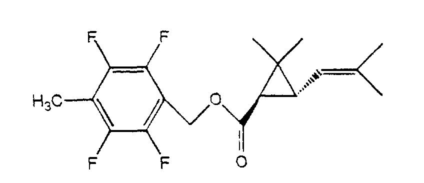

2. A pesticidal composition which comprises 2,3,5,6-tetrafluoro-4-methylbenzyl(1R)-trans-3-(2-methyl-1-propenyl)-2,2-dimethylcyclopropanecarboxylate as an active ingredient and a carrier.

3. A pesticidal composition for household use which comprises 2,3,5,6-tetrafluoro-4-methylbenzyl (1R)-trans-3-(2-methyl-1-propenyl)-2,2-dimethylcyclopropanecarboxylate as an active ingredient and a carrier.

4. A method for controlling pests which comprises applying 2,3,5,6-tetrafluoro-4-methylbenzyl (1R)-trans-3-(2-methyl-1-propenyl)-2,2-dimethylcyclopropanecarboxylate to a pest or a location which the pest inhabits.

5. A use of 2,3,5,6-tetrafluoro-4-methylbenzyl (1R)-trans-3-(2-methyl-1-propenyl)-2,2-dimethylcyclopropanecarboxylate as a pesticide.

6. The use according to claim 5 which takes place indoors.

## Patentansprüche

1. 2,3,5,6-Tetrafluor-4-methylbenzyl(1R)-trans-3-(2-methyl-1-propenyl)-2,2-dimethylcyclopropancarboxylat der Formel:

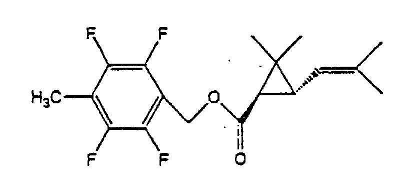

2. Pestizid-Zusammensetzung, welche 2,3,5,6-Tetrafluor-4-methylbenzyl(1R)-trans-3-(2-methyl-1-propenyl)-2,2-dimethylcyclopropancarboxylat als Wirkstoff und einen Träger umfaßt.

3. Pestizid-Zusammensetzung zur Verwendung im Haushalt, welche 2,3,5,6-Tetrafluor-4-methylbenzyl(1R)-trans-3-(2-methyl-1-propenyl)-2,2-dimethylcyclopropancarboxylat als Wirkstoff und einen Träger umfaßt.

4. Verfahren zur Bekämpfung von Schädlingen, welches das Aufbringen von 2,3,5,6-Tetrafluor-4-methylbenzyl(1R)-trans-3-(2-methyl-1-propenyl)-2,2-dimethylcyclopropancarboxylat auf einen Schädling oder einen Ort, der von einem Schädling befallen ist, umfaßt.

5. Verwendung von 2,3,5,6-Tetrafluor-4-methylbenzyl(1R)-trans-3-(2-methyl-1-propenyl)-2,2-dimethylcyclopropancarboxylat als Pestizid.

6. Verwendung nach Anspruch 5, welche in geschlossenen Räumen stattfindet.

## Revendications

1. 2,3,5,6 - tétrafluoro - 4 - méthylbenzyl (1R) - trans - 3 - (2 - méthyl - 1 - propényl) - 2,2 -diméthylcyclopropanecarboxylate illustré par la formule :

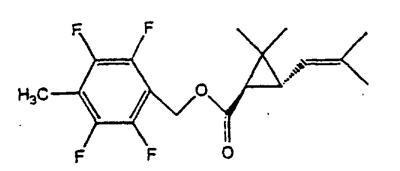

2. Composition pesticide comprenant du 2,3,5,6 - tétrafluoro - 4 - méthylbenzyl (1R) - trans - 3 - (2 - méthyl - 1 - propényl) - 2,2 - diméthylcyclopropanecarboxylate en tant qu'ingrédient actif, et un matériau formant support.

3. Composition pesticide à usage domestique comprenant du 2,3,5,6 - tétrafluoro - 4 - méthylbenzyl (1R) - trans - 3 - (2 - méthyl - 1 - propényl) - 2,2 -diméthylcyclopropanecarboxylate en tant qu'ingrédient actif, et un matériau formant support.

4. Procédé de contrôle des insectes comprenant le fait d'appliquer du 2,3,5,6 - tétrafluoro- 4- méthylbenzyl (1R) - trans - 3 - (2 - méthyl - 1 - propényl) - 2,2 -diméthylcyclopropanecarboxylate sur un insecte ou sur un endroit où vit l'insecte.

5. Utilisation de 2,3,5,6 - tétrafluoro - 4 - méthylbenzyl (1R) - trans - 3 - (2 - méthyl - 1 - propényl)- 2,2 -diméthylcyclopropanecarboxylate en tant que pesticide.

6. Utilisation selon la revendication 5, ayant lieu à l'intérieur.
